(19) Europäisches Patentamt
European Patent Office
Office européen des brevets

(11) **EP 0 993 866 A1**

(12) **EUROPÄISCHE PATENTANMELDUNG**

(43) Veröffentlichungstag:
19.04.2000 Patentblatt 2000/16

(51) Int. Cl.[7]: **B01J 23/58**, B01J 21/18,
C07C 45/00, B01J 37/02

(21) Anmeldenummer: 99119097.6

(22) Anmeldetag: 04.10.1999

(84) Benannte Vertragsstaaten:
**AT BE CH CY DE DK ES FI FR GB GR IE IT LI LU
MC NL PT SE**
Benannte Erstreckungsstaaten:
**AL LT LV MK RO SI**

(30) Priorität: 16.10.1998 DE 19847792

(71) Anmelder: **BAYER AG
51368 Leverkusen (DE)**

(72) Erfinder:
• **Pennemann, Bernd, Dr.
51467 Bergisch Gladbach (DE)**
• **Jentsch, Joerg-Dietrich
47799 Krefeld (DE)**
• **Schulze Tilling, Andreas, Dr.
51373 Leverkusen (DE)**
• **Wambach, Christoph
53937 Schleiden (DE)**
• **Wandelt, Klaus, Prof.
53913 Swisttal-Buschhoven (DE)**

(54) **Verfahren zur Herstellung von edelmetallhaltigen Katalysatoren auf Kohlenstoff-haltigen Trägermaterialen**

(57)     Bereitgestellt wird ein Verfahren zur Herstellung von edelmetallhaltigen Katalysatoren, die als katalytisch aktive Komponente mindestens ein Edelmetall der Platinmetallgruppe und/oder dessen Verbindungen sowie gegebenenfalls Promotoren und/oder Modifikatoren auf einem Kohlenstoff-haltigen Trägermaterial enthalten. Bei diesem Verfahren wird das Kohlenstoffhaltige Trägermaterial vor dem Aufbringen der katalytisch aktiven Edelmetall-Komponenten sowie gegebenenfalls Promotoren und/oder Modifikatoren mit Alkalioder Erdalkalimetallsalzen organischer oder anorganischer Säuren behandelt. Die erhaltenen Katalysatoren lassen sich mit Erfolg insbesondere bei der Herstellung von gegebenenfalls substituierten Cyclohexanonen durch Hydrierung der entsprechend substituierten Phenole einsetzen.

**Beschreibung**

Die vorliegende Erfindung betrifft ein Verfahren zur Herstellung von edelmetallhaltigen Katalysatoren auf einem Kohlenstoff-haltigen Trägermaterial, die dabei erhaltenen Katalysatoren sowie deren Verwendung zur Herstellung von substituierten Cyclohexanonen.

Die Herstellung von organischen Verbindungen wird großtechnisch in Gegenwart von edelmetallhaltigen Katalysatoren durchgeführt. Ein wichtiges Anwendungsgebiet sind beispielsweise Hydrierreaktionen. Aktivität und Selektivität solcher Katalysatoren können durch zusätzliche Promotoren und Modifikatoren beeinflußt werden. Promotoren verstärken die katalytische Wirkung, während Modifikatoren die Selektivität durch teilweise Vergiftung des Katalysators verändern. Je nach gewünschter Reaktion können bestimmte Komponenten in einem Fall als Promotoren und im anderen Fall als Modifikatoren wirken. In der Regel werden die katalytisch aktiven Edelmetalle auf ein inertes Trägermaterial aufgebracht. Gegenüber nicht trägerfixierten Edelmetallkatalysatoren weisen Trägerkatalysatoren eine erhöhte thermische und mechanische Beständigkeit auf und führen zu einer deutlich erhöhten aktiven Oberfläche der entsprechenden Edelmetalle.

Kohlenstoff-haltige Trägermaterialien gewinnen in der Katalyse zunehmend an Bedeutung. Dies liegt zum einen an der nahezu unbegrenzten Verfügbarkeit und dem günstigen Preis und zum anderen an den katalytisch vorteilhaften Eigenschaften. So besitzen die für den katalytischen Einsatz geeigneten Graphite, Aktivkohlenstoffe und Ruße eine hohe Resistenz gegenüber Säuren und Basen, hohe Schmelzpunkte und z.T. sehr gute elektrische Leitfähigkeiten. Von Vorteil ist ferner, daß die physikalischen und chemischen Eigenschaften wie die Porosität oder die vorhandenen fünktionellen Oberflächengruppen in weiten Bereichen modifiziert werden können.

Insbesondere Aktivkohlenstoffe haben sich als Trägermaterialien für edelmetallhaltige Katalysatoren bewährt. Es handelt sich bei den Aktivkohlenstoffen um hochporöse, feinkristalline Substanzen, die mikrokristalline, dem Graphitgitter entnommene Bereiche aufweisen, die überwiegend fehlgeordnet und gegeneinander verdreht vorliegen. Aktivkohlenstoffe werden durch Verkohlung mit nachfolgender chemischer Aktivierung oder Gasaktivierung aus kohlenstoffhaltigen Materialien wie Holz, Nußschalen, Fruchtkernen, Torf, Holz-, Stein- oder Braunkohle sowie sonstigen Erdölprodukten gewonnen. Durch die im Aktivierungsschritt erfolgende Entfernung von amorphem Kohlenstoff kommt es zur Ausbildung eines Porensystems und damit zu einer Vergrößerung der spezifischen Oberfläche. Es entstehen Aktivkohlen mit einem Porenvolumen von bis zu 1,5 ml/g und einer spezifischen Oberfläche von bis zu 2500 $m^2$/g. Letztere kann aus den Stickstoff-Adsorptionsisothermen über die Auswertung nach der Methode von Brunauer, Emmett und Teller (BET) bestimmt werden (DIN 66132). Aktivkohlenstoffe enthalten neben Kohlenstoff geringe Mengen an Sauerstoff und Wasserstoff, die in Form verschiedener funktioneller Gruppen, wie z.B. Phenol-, Carbonyl-, Carboxyl-, Lacton-, Chinon- und Ethergruppen vorliegen. Welche funktionellen Gruppen in welchen Mengen auf der Aktivkohlenoberfläche vorhanden sind, wird durch das Rohmaterial und den Aktivierungsprozeß bestimmt. Aktivkohlen enthalten ferner eine Reihe mineralischer Bestandteile, wie Kieselsäuren sowie Alkali- und Erdalkalisalze. Deren Gehalt wird von den Herstellern unter dem Begriff „Aschegehalt" angegeben.

Die Herstellung von palladiumhaltigen Schalenkatalysatoren auf Aktivkohle als Träger wird in US-A-3.271.327 beschrieben. Hierbei wird zunächst eine wässrige Lösung von Palladiumchlorid und Salzsäure durch Zugabe einer Base auf einen pH-Wert von 4 bis 6,5 eingestellt. Diese Lösung wird dann mit dem Trägermaterial in Kontakt gebracht, für eine gewisse Zeit der Imprägnierung bei einer Temperatur von ca. 21-30°C gehalten und anschließend bei Temperaturen unterhalb von 60°C getrocknet. Eine Reduktion der Katalysatoren vor ihrem Einsatz in Hydrierreaktionen ist nicht nötig, ihre Aktivierung erfolgt vielmehr in situ während der katalysierten Hydrierungen durch den anwesenden Wasserstoff Die so hergestellten Katalysatoren besitzen jedoch im Hinblick auf ihre Aktivität sowie das Selektivitätsprofil der katalysierten Reaktionen noch Verbesserungspotential.

Der Einsatz von palladiumhaltigen Schalenkatalysatoren bei der Hydrierung von substituierten oder nicht substituierten Phenolen zur Herstellung der entsprechenden Cyclohexanone ist bekannt. So wird z.B. in J. Mol. Cat. 55 (1989) 415-428 die Herstellung von 2,6-Dimethylanilin aus 2,6-Dimethylphenol unter Verwendung eines palladiumhaltigen Katalysators in Gegenwart von Ammoniak beschrieben. Hierbei wird zunächst 2,6-Dimethylcyclohexanon erhalten, welches dann mit Ammoniak weiter reagiert. Bei dem verwendeten palladiumhaltigen Schalenkatalysator handelt es sich um kolloidales Palladiumhydroxid auf Sibunit Kohlenstoff als Trägermaterial. Zur Herstellung dieses Katalysators wird eine wässrige Natriumcarbonat-Lösung mit einer wässrigen Lösung von $H_2PdCl_2$ vereinigt, wobei kolloidales Palladiumhydroxid entsteht. Diese Lösung wird anschließend zu dem porösen Trägermaterial Sibunit gegeben.

Aufgrund der steigenden Bedeutung edelmetallhaltiger Katalysatoren für Hydrierreaktionen bestand die Aufgabe der vorliegenden Erfindung darin, edelmetallhaltige Trägerkatalysatoren für Verfügung zu stellen, die in Hydrierreaktionen, insbesondere der Hydrierung von gegebenenfalls substituierten Phenolen, zu verbesserten Ergebnissen führen.

Gegenstand der vorliegenden Erfindung ist ein Verfahren zur Herstellung von edelmetallhaltigen Katalysatoren, die als katalytisch aktive Komponente mindestens ein Edelmetall der Platinmetallgruppe und/oder dessen Verbindungen sowie gegebenenfalls Promotoren und/oder Modifikatoren auf einem Kohlenstoff-haltigen Trägermaterial enthalten, welches dadurch gekennzeichnet ist, daß das Kohlenstoff-haltige Trägermaterial zunächst mit Alkali- oder Erdalkalimetallsalzen organischer oder anorganischer Säuren behandelt wird und anschließend die katalytisch aktiven Edelmetall-Komponenten sowie gegebenenfalls Promotoren und/oder Modifikatoren auf das behandelte Kohlenstoff-haltige Trägermaterial aufgebracht werden.

Mit dem erfindungsgemäßen Verfahren werden Katalysatoren erhalten, die beim Einsatz in der Hydrierung von gegebenenfalls substituierten Phenolen zu den entsprechenden Cyclohexanonen exzellente Aktivitäts- und Selektivitätswerte liefern. Wesentlich ist im erfindungsgemäßen Verfahren, daß das Kohlenstoff-haltige Trägermaterial vor dem Aufbringen der katalytisch aktiven Edelmetall-Komponenten sowie gegebenenfalls Promotoren und/oder Modifikatoren mit Alkali- oder Erdalkalimetallsalzen organischer oder anorganischer Säuren behandelt wird. Bewährt haben sich hierbei insbesondere die Natrium- und Kaliumsalze organischer oder anorganischer Säuren. Als organische Säuren wird bevorzugt Ameisensäure und als anorganische Säuren werden bevorzugt Halogenwasserstoffsäuren, insbesondere Salzsäure, oder Salpetersäure eingesetzt. Besonders bevorzugt sind Natriumformiat, Natriumchlorid und Natriumnitrat. Die eingesetzten Salzlösungen besitzen üblicherweise eine Konzentration von 1-100 mmol/l Lösung.

Als Kohlenstoff-haltiges Trägermaterial können im erfindungsgemäßen Verfahren beispielsweise Graphite, Aktivkohlen oder Ruße verwendet werden. Bevorzugt setzt man Aktivkohle ein, wobei diese üblicherweise pulverförmig, d.h. in feinteiliger Form mit einem mittleren Teilchendurchmesser von 15 bis 30 μm, oder als stückige Formkörper mit Abmessungen von 1 bis 5 mm zur Anwendung kommen. Sie besitzen in der Regel eine spezifische Oberfläche von bis zu 2500 $m^2$/g, bevorzugt 300 - 1200 $m^2$/g, ein Gesamtporenvolumen von mehr als 0,5 ml/g und einen Restaschegehalt von <5 Gew.-%. Die Aktivkohle kann dabei im unbehandelten Zustand eingesetzt werden, es hat sich jedoch in vielen Fällen bewährt, eine Vorbehandlung durchzuführen. Eine solche Vorbehandlung umfaßt gewöhnlich das Waschen der Aktivkohle mit starken Mineralsäuren wie z.B. Salzsäure oder Salpetersäure. Hierdurch wird der teilweise hohe Aschegehalt kommerzieller Aktivkohle (bis zu 20 %), der von den mineralischen Komponenten der Ausgangsmaterialien herrührt, deutlich verringert. Entfernt werden erdalkali- und schwermetallhaltige Verbindungen. Zum anderen wird durch den Waschvorgang auch die Trägeroberfläche neu funktionalisiert. Beides wirkt sich vorteilhaft auf die Aktivität des fertigen Katalysator aus.

Nach einer solchen Wäsche liegen die Restaschegehalte der eingesetzten Aktivkohle typischerweise unter 2 Gew.-%. Gemäß DE-OS- 43 08 101 kann es ferner vorteilhaft sein, den mit Salpetersäure gewaschenen Aktivkohleträger vor der Zugabe der Salze und dem Aufbringen des Edelmetalls einer oxidativen Vorbehandlung mit Wasserstoffperoxid oder Natriumhypochlorit zu unterziehen. Diese oxidative Vorbehandlung wird bevorzugt in Form einer Wäsche mit einer 0,1 bis 30 Gew.-% Wasserstoffperoxid oder Natriumhypochlorit enthaltenden wässrigen Lösung bei Raumtemperatur für die Dauer von 100 bis 300 Minuten durchgeführt.

Die Durchführung des erfindungsgemäßen Verfahrens kann in verschiedenen Ausführungsformen erfolgen.

In einer Ausführungsform A geht man so vor, daß man

1) eine 10 - 70 %ige, bevorzugt 50 %ige Suspension des Kohlenstoff-haltigen Trägermaterials in Wasser herstellt,
2) hierzu die wässrige Lösung eines Alkali- oder Erdalkalisalzes einer organischen oder anorganischen Säure gibt,
3) einen pH-Wert im Bereich von 1 bis 6,5, bevorzugt 2 bis 6, einstellt,
4) zu dieser Suspension eine wässrige Lösung der wasserlöslichen Edelmetallverbindungen gibt, die zuvor bevorzugt auf den gleichen pH-Wert eingestellt wurde wie die Suspension des Kohlenstoff-haltigen Trägermaterials, und
5) den Katalysator abfiltriert und trocknet.

In diesem Fall enthält der Katalysator die katalytisch aktive Edelmetall-Komponente in Form der entsprechenden Kationen auf dem Kohlenstoff-haltigen Trägermaterial, im Fall von Palladium somit als $Pd^{2+}$ Ionen.

In einer zweiten Ausführungsform B geht man so vor, daß man im Anschluß an die Schritte 1) bis 4) der Ausführungsform A

6) die wasserlöslichen Edelmetallverbindungen durch Zugabe einer Base in schwer oder wasserunlösliche Verbindungen überführt und
7) den Katalysator abfiltriert, wäscht und trocknet.

Bei beiden Ausführungsformen A und B kann der erhaltene Katalysator ohne weitere Reduktion als Katalysator in Hydrierreaktionen eingesetzt werden, wo dann in situ durch den anwesenden Wasserstoff die Aktivierung erfolgt.

In einer dritten Ausführungsform C geht man so vor, daß man im Anschluß an die Schritte 1) bis 4) der Ausführungsform A

8) eine Reduktion der Edelmetallverbindungen durchführt und
9) den Katalysator abfiltriert, wäscht und trocknet.

Die Einstellung des pH-Wertes der Suspension des Kohlenstoff-haltigen Trägermaterials und der wässrigen Lösung der Edelmetallverbindungen erfolgt durch Zugabe von wässrigen anorganischen Säuren, beispielsweise HCl, oder wässrigen Basen, beispielsweise Alkalimetallhydroxiden, insbesondere NaOH. Die wässrige Lösung der Edelmetallverbindungen wird üblicherweise unter Rühren und pH-Wert Kontrolle zu der Suspension des Kohlenstoff-haltigen Trägermaterials gegeben. Bevorzugt wird der pH-Wert ungefähr auf dem Wert konstant gehalten, den auch die beiden Ausgangslösungen, d.h. die Suspension des Trägermaterials und die edelmetallhaltige Lösung, vorher besitzen. Die Reaktionstemperatur liegt üblicherweise im Bereich von 20 bis 90°C, bevorzugt 20 bis 50°C. Die Reaktionszeit beträgt dabei von 1 Minute bis zu 24 Stunden, bevorzugt 60 bis 90 Minuten.

In Schritt 8 der Ausführungsform C werden die Edelmetallkationen in das entsprechende Edelmetall überführt. Als Reduktionsmittel können Hydrazin, Natriumformiat, Natriumboranat, Formaldehyd oder Wasserstoff eingesetzt werden. Die Reduktionstemperatur liegt im allgemeinen bei 0 bis 100 °C, vorzugsweise bei 0 bis 50°C. Wird als Reduktionsmittel ein Gas verwendet, so hat es sich bewährt, dieses zunächst mit einem Inertgas, wie Stickstoff, Kohlendioxid oder einem Edelgas, zu verdünnen. Die Reduktion kann auch in flüssiger Phase bei einer Temperatur von 0 bis 50°C, vorzugsweise 0 bis 25 °C durchgeführt werden. Als Reduktionsmittel lassen sich wassrige Lösungen von Hydrazin, Ameisensäure oder Alkaliborhydriden verwenden. Bevorzugt erfolgt die Reduktion mittels Formaldehyd in wässrigem alkalischen Medium, insbesondere bei einem pH-Wert von 8 bis 9, und bei Temperaturen von 0 bis 10°C.

Als katalytisch aktive Edelmetalle aus der Platinmetallgruppe haben sich Platin, Palladium, Rhodium, Ruthenium, Osmium und Iridium bzw. Kombinationen von diesen bewährt. Besonders bevorzugt sind Platin, Palladium, Rhodium und Ruthenium, insbesondere wird Palladium verwendet. Die Platinmetalle werden im erfindungsgemäßen Verfahren in Form wasserlöslicher Verbindungen eingesetzt. Bewährt haben sich hierbei die leicht zugängliche Tetrachloropalladatsäure, die Hexachloroplatinsäure, Palladiumnitrat, Palladiumoxidhydrat und Palladiumacetylacetonat sowie Amminkomplexe des Palladiums. Tetrachloropalladatsäure und Hexachloroplatinsäure können durch Zugabe von konzentrierter Salzsäure zu Palladium(II)chlorid bzw. Platin(IV)chlorid hergestellt werden, so daß eine 0,1 bis 5 M, bevorzugt 1 bis 1,5 M wässrige Lösung entsteht. Der Edelmetallgehalt des Katalysators liegt bei 0,1 bis 20 Gew.-%, bevorzugt 0,1 bis 10 Gew.-%, bezogen auf das Kohlenstoff-haltige Trägermaterial.

Sofern der Katalysator auch Promotoren und/oder Modifikatoren enthalten soll, werden wasserlösliche Verbindungen dieser Promotoren und/oder Modifikatoren der wässrigen Lösung der Edelmetallverbindungen zugesetzt. Bekannte Promotoren bzw. Modifikatoren sind beispielsweise die Edelmetalle Au, Ag, Hg oder Ru sowie S, Pb, Bi, Cu, Fe oder Ni.

Gegenstand der Erfindung sind ferner die über das erfindungsgemäße Verfahren erhältlichen Katalysatoren und die Verwendung dieser Katalysatoren in Hydrierreaktionen, bevorzugt bei der Hydrierung von gegebenenfalls substituierten Phenolen zu den entsprechenden Cyclohexanonen.

Die Hydrierung der gegebenenfalls substituierten Phenole mit Wasserstoff zu den entsprechenden Cyclohexanonen wird üblicherweise in der Sumpfphase in einer dem Fachmann bekannten Weise bei einer Reaktionstemperatur von 100 bis 180°C, bevorzugt 140 bis 160°C, und einem Druck von 0,1 bis 5 MPa, bevorzugt 0,5 bis 2 MPa, durchgeführt. Der erfindungsgemäße Katalysator wird dabei in einer Menge von 0,1 bis 5, bevorzugt 0,5 bis 2 und insbesondere 0,75 bis 1 Gew.-%, bezogen auf das eingesetzte Phenol, verwendet. Die Reaktion wird in herkömmlichen Autoklaven durchgeführt. Die Hydrierung kann auch in der Gasphase z.B. in einem Strömungsrohr bei einer Reaktionstemperatur von 100 bis 180°C, bevorzugt 140 bis 160°C, und einem Druck von 0,1 bis 0,5 MPa erfolgen. Wasserstoff und Phenol werden dabei in einem molaren Verhältnis von (2-5):1, bevorzugt (3-5):1 eingesetzt. Ein noch größerer Wasserstoff-Überschuß ist möglich, führt aber infolge einer stärkeren Bildung des entsprechenden Cyclohexanols zu einer schlechteren Selektivität. Die zu hydrierenden Phenole können durch einen oder mehrere Reste, die sich in der Hydrierung inert verhalten, substituiert sein. Beispiele hierfür sind Halogenreste sowie geradkettige oder verzweigte $C_1$-$C_8$-Alkylreste, bevorzugt Methyl-, Ethyl-, Propyl-, i-Propyl, n-Butyl, i-Butyl oder t-Butylreste.

### Beispiel 1 (Herstellung von Katalysator 1)

10 g Aktivkohle werden bei Raumtemperatur in 80 ml Wasser suspendiert und nach Zusatz von 4 mmol Natriumformiat durch Zusatz von HCl auf einen pH-Wert von 2 eingestellt (Lösung 1). In einem zweiten Gefäß wird die Lösung 2 aus 3,6 ml einer 1 M Tetrachloro-palladatsäure und 40 ml Wasser durch Zusatz von NaOH ebenfalls auf einen pH Wert von 2 eingestellt. Anschließend gibt man unter starkem Rühren schnell Lösung 2 zu Lösung 1 und korrigiert Abweichungen vom pH-Wert 2. Nach einer Stunde wird unter weiterem Rühren die Reaktionslösung mittels eines Kühlbades auf 0°C abgekühlt, 0,51 mL einer 36,5 %igen Formaldehyd Lösung zugesetzt und mit NaOH schnell ein pH-Wert von >8 eingestellt. Nach Entfernen des Kühlbades läßt man die Lösung noch so lange rühren, bis wieder Raumtemperatur erreicht wird. Anschließend filtriert man den erhaltenen Katalysator ab und trocknet ihn im Vakuum über einem Trockenmittel.

### Vergleichsbeispiel 1 (Herstellung von Katalysator V1)

Das Vergleichsbeispiel 1 wird analog zu Beispiel 1 durchgeführt, allerdings wird der Aktivkohle nach der Suspendierung in Wasser kein Natriumformiat zugegeben.

### Beispiel 2 (Herstellung von Katalysator 2)

Die Herstellung des Katalysators 2 erfolgt analog zur Herstellung des Katalysators 1, allerdings wird bei den Lösungen 1 und 2 jeweils ein pH-Wert von 6 eingestellt und dieser Wert auch bei Zugabe von Lösung 2 zu Lösung 1 weitgehend konstant gehalten.

### Vergleichsbeispiel 2 (Herstellung von Katalysator V2)

Die Herstellung des Katalysators V2 erfolgt analog zur Herstellung des Katalysators V1, allerdings wird bei den Lösungen 1 und 2 jeweils ein pH-Wert von 6 eingestellt.

### Beispiele 3 und 4 und Vergleichsbeispiele V3 und V4 (Hydrierung von Phenol)

Die Leistungen der Katalysatoren 1,2, V1 und V2 werden in einer Sumpfphasen-Mitteldruckhydrierung von Phenol zu Cyclohexanon untersucht.

Jeweils 2,25 g des Katalysators werden zusammen mit 300 g verflüssigtem Phenol in einen auf 80°C vorgeheizten 1 l Autoklaven eingebracht und anschließend bei stehendem Rührer unter Stickstoffatmosphäre auf 140°C erhitzt. Nach Erreichen der Reaktionstemperatur wird Stickstoff durch Wasserstoff ersetzt und ein Reaktordruck von 5 bar eingestellt. Der Reaktionsbeginn wird durch das Einschalten des Rührers (Rührerdrehzahl 1000 U/min) gekennzeichnet. Verfolgt wird die Wasserstoffaufnahme während der Reaktion durch den abnehmenden Gasdruck in zwei Wasserstoffvorratsbehältern, die eine konstante Gaszulieferung gewährleisten. Bei Erreichen einer Gesamtwasserstoffaufnahme von 98,5 % wird die Reaktion durch Anhalten des Rührers und Kühlung des Autoklaven abgebrochen.

Als Reaktionszeitraum für die Berechnung der Aktivität dient die Reaktionszeit zwischen der 15 und 20. Minute. Die Angabe der Aktivität erfolt in

$$[\, l\, [H_2]/\, g_{Kat.} * min$$

$$[t1/2_{\pm} 2.5\ min]].$$

Die Selektivität ergibt sich als Verhältnis der Geschwindigkeitskonstanten $k_1/k_2$, wobei $k_1$ die Reaktion des Phenols zu Cyclohexanon und $k_2$ die Folgereaktion von Cyclohexanon zu Cyclohexanol charakterisiert.

Die Ergebnisse der Hydrierungen sind in der nachfolgenden Tabelle 1 zusammengefaßt.

| Beispiel | 3 | V3 | 4 | V4 |
|---|---|---|---|---|
| Katalysator | 1 (Synthese bei pH=2 mit Formiat) | V1 (Synthese bei pH=2) | 2 (Synthese bei pH=6 mit Formiat) | V2 (Synthese bei pH=6) |
| Reaktionsprodukt: | | | | |
| Phenol [%] | 0 | 0 | 0 | 0 |
| Cyclohexanon [%] | 88.2 | 79 | 94.5 | 89.6 |
| Cyclohexanol [%] | 11.6 | 20 | 5.4 | 10.2 |
| | | | | |
| Selektivität ($k_1/k_2$) | 36.58 | 10.63 | 62.0 | 30.38 |
| Aktivität | 0.66 | 0.11 | 1.0 | 0.97 |
| $t_{ges}$ [min] | 328 | 649 | 187.4 | 257 |

Die durchgeführten Versuche zur Hydrierung von Phenol zu Cyclohexanon belegen, daß die nach dem erfindungsgemäßen Verfahren unter Zusatz von Natriumformiat hergestellten Katalysatoren im Vergleich zu den ohne Salzzugabe hergestellten Katalysatoren eine deutlich höhere Aktivität und Selektivität aufweisen und gleichzeitig eine stark verkürzte Reaktionszeit ermöglichen.

**Patentansprüche**

1. Verfahren zur Herstellung von edelmetallhaltigen Katalysatoren, die als katalytisch aktive Komponente mindestens ein Edelmetall der Platinmetallgruppe und/oder dessen Verbindungen sowie gegebenenfalls Promotoren und/oder Modifikatoren auf einem Kohlenstoff-haltigen Trägermaterial enthalten, dadurch gekennzeichnet, daß das Kohlenstoff-haltige Trägermaterial zunächst mit Alkali- oder Erdalkalimetallsalzen organischer oder anorganischer Säuren behandelt wird und anschließend die katalytisch aktiven Edelmetall-Komponenten sowie gegebenenfalls Promotoren und/oder Modifikatoren auf das behandelte Kohlenstoff-haltige Trägermaterial aufgebracht werden.

2. Verfahren nach Anspruch 1, dadurch gekennzeichnet, daß das Kohlenstoffhaltige Trägermaterial mit Natrium- oder Kaliumsalzen organischer Säuren, bevorzugt Ameisensäure, oder anorganischer Säuren, bevorzugt Halogenwasserstoffsäuren, insbesondere Salzsäure, oder Salpetersäure behandelt wird.

3. Verfahren nach Anspruch 2, dadurch gekennzeichnet, daß Natriumformiat, Natriumchlorid oder Natriumnitrat eingesetzt wird.

4. Verfahren nach Anspruch 1, dadurch gekennzeichnet, daß man

   1) eine 10 - 70 %ige Suspension des Kohlenstoff-haltigen Trägermaterials in Wasser herstellt,
   2) hierzu die wässrige Lösung eines Alkali- oder Erdalkalisalzes einer organischen oder anorganischen Säure gibt,
   3) einen pH-Wert im Bereich von 1-6.5 einstellt,
   4) zu dieser Suspension eine wässrige Lösung der wasserlöslichen Edelmetallverbindungen gibt, die zuvor bevorzugt auf den gleichen pH-Wert eingestellt wurde wie die Suspension des Kohlenstoff-haltigen Trägermaterials, und
   5) den Katalysator abfiltriert und trocknet.

5. Verfahren nach Anspruch 4, dadurch gekennzeichnet, daß man im Anschluß an die Schritte 1) bis 4)

   6) die wasserlöslichen Edelmetallverbindungen durch Zugabe einer Base in schwer oder wasserunlösliche Verbindungen überführt und
   7) den Katalysator abfiltriert, wäscht und trocknet.

6. Verfahren nach Anspruch 4, dadurch gekennzeichnet, daß man im Anschluß an die Schritte 1) bis 4)

   8) eine Reduktion der Edelmetallverbindungen durchführt und
   9) den Katalysator abfiltriert, wäscht und trocknet.

7. Verfahren nach einem oder mehreren der Ansprüche 1 bis 6, dadurch gekennzeichnet, daß als Edelmetall aus der Platinmetallgruppe Platin, Palladium, Rhodium, Ruthenium, Osmium oder Iridium oder deren Kombinationen verwendet wird.

8. Verfahren nach einem oder mehreren der Ansprüche 1 - 7, dadurch gekennzeichnet, daß als Kohlenstoff-haltiges Trägermaterial Aktivkohle verwendet wird.

9. Edelmetallhaltige Katalysatoren erhältlich nach dem Verfahren gemäß einem oder mehreren der Ansprüche 1 bis 8.

10. Verwendung der Katalysatoren nach Anspruch 9 in Hydrierreaktionen.

11. Verwendung der Katalysatoren nach Anspruch 10 bei der Herstellung von gegebenenfalls substituierten Cyclohexanonen durch Hydrierung der entsprechend substituierten Phenole.

12. Verwendung nach Anspruch 11, wobei die Phenole durch einen oder mehrere Halogenreste oder geradkettige oder verzweigte $C_1$-$C_8$-Alkylreste, bevorzugt Methyl-, Ethyl-, Propyl-, i-Propyl, n-Butyl, i-Butyl oder t-Butylreste substituiert sind.

Europäisches
Patentamt

**EUROPÄISCHER RECHERCHENBERICHT**

Nummer der Anmeldung

EP 99 11 9097

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | US 4 200 553 A (FISHER WILLIAM B ET AL) 29. April 1980 (1980-04-29) * das ganze Dokument * | 9-11 | B01J23/58 B01J21/18 C07C45/00 B01J37/02 |
| X | EP 0 054 158 A (WACKER CHEMIE GMBH) 23. Juni 1982 (1982-06-23) * das ganze Dokument * | 9,10 | |
| A | | 1-4,7,8 | |
| X | US 4 659 686 A (GRIFFITHS BONNIE W ET AL) 21. April 1987 (1987-04-21) | 9,10 | |
| A | * Ansprüche 3,4 * * Beispiele 48-56 * | 1-4,7,8 | |
| X | US 3 663 166 A (WEISE JOHANNES ET AL) 16. Mai 1971 (1971-05-16) | 9,10 | |
| A | * Beispiele 1,2 * | 1-4,7,8 | |
| D,X | US 3 271 327 A (MCEVOY JAMES E ET AL) 6. September 1966 (1966-09-06) * das ganze Dokument * | 9,10 | **RECHERCHIERTE SACHGEBIETE (Int.Cl.7)** B01J C07C |

Der vorliegende Recherchenbericht wurde für alle Patentansprüche erstellt

| Recherchenort | Abschlußdatum der Recherche | Prüfer |
|---|---|---|
| DEN HAAG | 24. Januar 2000 | Zuurdeeg, B |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

EP 0 993 866 A1

## ANHANG ZUM EUROPÄISCHEN RECHERCHENBERICHT
## ÜBER DIE EUROPÄISCHE PATENTANMELDUNG NR.

EP 99 11 9097

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

24-01-2000

| Im Recherchenbericht angeführtes Patentdokument | Datum der Veröffentlichung | Mitglied(er) der Patentfamilie | Datum der Veröffentlichung |
|---|---|---|---|
| US 4200553 A | 29-04-1980 | KEINE | |
| EP 0054158 A | 23-06-1982 | DE 3047347 A | 22-07-1982 |
| | | JP 1235945 C | 17-10-1984 |
| | | JP 57102232 A | 25-06-1982 |
| | | JP 59012341 B | 22-03-1984 |
| | | US 4415499 A | 15-11-1983 |
| US 4659686 A | 21-04-1987 | KEINE | |
| US 3663166 A | 16-05-1972 | BE 715052 A | 13-11-1968 |
| | | CH 498652 A | 15-11-1970 |
| | | DE 1667045 A | 03-06-1971 |
| | | FR 1574146 A | 11-07-1969 |
| | | GB 1229493 A | 21-04-1971 |
| | | NL 6806597 A | 14-11-1968 |
| US 3271327 A | 06-09-1966 | KEINE | |

Für nähere Einzelheiten zu diesem Anhang : siehe Amtsblatt des Europäischen Patentamts, Nr.12/82

8